# FASCICULE DE BREVET EUROPEEN

(11) **EP 1 224 001 B1**
(45) Date de publication et mention de la délivrance du brevet: **17.12.2003**
(21) Numéro de dépôt: 00974570.4
(22) Date de dépôt: 26.10.2000
(51) Int. Cl.: A61M 5/32

(54) **SERINGUE HYPODERMIQUE A JETER AVEC FOURREAU DE PROTECTION VERROUILLABLE**
HYPODERMATISCHE WEGWERFSPRITZE MIT ARRETIERBARER SCHUTZHÜLSE
DISPOSABLE HYPODERMIC SYRINGE WITH LOCKING PROTECTIVE SHEATH

(30) Priorité: 26.10.1999 FR 9913347
(43) Date de publication de la demande: 24.07.2002
(73) Titulaire: COMPAGNIE PLASTIC OMNIUM, 69007 Lyon (FR)
(72) Inventeur: POUGET, Michel, F-38300 Domarin (FR); BONACCI, Fabrice, F-69800 Saint Priest (FR)
(74) Mandataire: Leszczynski, André
(86) Numéro de dépôt international: FR0002987
(87) Numéro de publication internationale: WO01030428

(56) Documents cités:
- EP-A- 0 680 767
- WO-A-93/00949
- US-A- 5 433 712
- US-A- 5 562 626

## Description

La présente invention concerne un dispositif de sécurité pour seringue qui est muni d'une tête télescopique.

On sait que certains produits médicamenteux injectables sont distribués par doses dans des seringues préremplies auxquelles il suffit d'ajouter une tige de piston et une aiguille pour pouvoir les utiliser.

On sait par ailleurs que les seringues usagées constituent un danger, notamment pour le personnel médical, car l'aiguille d'une seringue, après utilisation, est souillée et potentiellement contaminante pour des personnes qui entreraient en contact avec l'aiguille ou s'y piqueraient accidentellement.

On connaît par le brevet US 5 562 626 et la demande EP 0 687 167 un dispositif de sécurité pour une seringue comportant un fourreau apte à venir recouvrir l'aiguille de la seringue en fin d'injection.

La présente invention vise à fournir un dispositif de sécurité qui évite tout risque de blessure ou de piqûre avec l'aiguille d'une seringue ayant déjà servi à une injection de produit.

La présente invention a pour objet un dispositif de sécurité pour une seringue constituée d'un corps, d'un porte-aiguille monté à une extrémité de ce corps, d'un piston mobile dans le corps et d'une tige de piston dépassant du corps à l'opposé du porte-aiguille et apte à pousser le piston dans le corps en direction du porte-aiguille, le dispositif étant caractérisé par le fait qu'il comprend un fourreau ayant une extrémité avant et une extrémité arrière, dans lequel un corps de seringue peut coulisser axialement entre une position d'injection dans laquelle le corps de la seringue est intégralement contenu dans le fourreau, tandis que le porte-aiguille de la seringue affleure à l'extrémité avant du fourreau et une position de sécurité dans laquelle une partie du corps de la seringue dépasse de l'extrémité arrière du fourreau, tandis que le porte-aiguille se trouve en retrait de l'extrémité avant du fourreau, le dispositif comprenant également un organe de rappel du corps de seringue en position de sécurité, lequel organe de rappel se déclenche automatiquement lorsque l'on enfonce la tige de piston dans le corps de la seringue, le fourreau comportant, à son extrémité arrière, une tête télescopique pouvant prendre une position rentrée dans laquelle elle n'agit pas sur le corps de la seringue en position d'injection et une position sortie dans laquelle elle enveloppe la partie du corps de la seringue en position de sécurité dépassant de l'extrémité arrière du fourreau, le dispositif comportant en outre des moyens de verrouillage de la tête télescopique en position sortie, constitués par une combinaison de deux portées d'appui axiales opposées de la tête télescopique qui s'appuient sur deux portées d'appui axiales opposées du fourreau et de deux portées d'appui latérales opposées de la tête télescopique qui s'appuient sur deux portées d'appui latérales opposées du fourreau.

Grâce au dispositif selon l'invention, lorsque l'injection du produit est terminée, l'organe de rappel fait remonter le corps de la seringue dans le fourreau en position de sécurité, ce qui provoque le retrait de l'aiguille de la seringue à l'intérieur du fourreau.

Tout contact accidentel avec cette aiguille devient alors impossible.

Grâce à la tête télescopique du fourreau qui, en position sortie, enveloppe la partie du corps de seringue dépassant de l'extrémité arrière du fourreau, la seringue est retenue dans le fourreau et ne peut s'en échapper par son extrémité arrière.

Selon l'invention, la tête télescopique est fermement solidarisée au fourreau lorsqu'elle se trouve en position sortie grâce à l'appui mutuel des deux portées axiales opposées du fourreau sur les deux portées axiales opposées de la tête, en combinaison avec l'appui mutuel des deux portées latérales opposées du fourreau sur les deux portées latérales opposées de la tête.

Les portées axiales réalisent l'immobilisation axiale de la tête télescopique par rapport au fourreau.

Les portées latérales ont pour fonction de maintenir les portées axiales en appui, même en cas de légère déformation radiale de la tête du fourreau, afin que l'immobilisation axiale de la tête par rapport au fourreau ne soit pas rompue par une pression exercée latéralement sur la tête ou sur le fourreau.

En d'autres termes, dans le dispositif de sécurité selon l'invention, si la tête télescopique ou le fourreau vient à subir une contrainte perpendiculaire à l'axe du dispositif, la déformation radiale de la tête télescopique ou du fourreau qui en résulte ne provoque aucune désolidarisation axiale de la tête par rapport au fourreau.

Dans un mode de réalisation particulier de l'invention, le corps de la seringue est immobilisé axialement par rapport au fourreau par l'intermédiaire de la tête télescopique.

Dans ce mode de réalisation, le corps de la seringue est immobilisé en position de sécurité par rapport à la tête télescopique, laquelle est, comme on vient de le voir, fermement immobilisée axialement par rapport au fourreau, conformément à l'invention.

Dans un autre mode de réalisation de l'invention, le corps de la seringue est immobilisé en position de sécurité par rapport au fourreau par appui direct du corps de la seringue sur une portée d'appui du fourreau.

Dans un mode de réalisation particulier de l'invention, la tête du fourreau sert à déclencher l'organe de rappel lorsque l'on enfonce la tige de piston dans le corps de la seringue.

Dans le but de mieux faire comprendre l'invention, on va en décrire maintenant un mode de réalisation donné à titre d'exemple non limitatif, en référence au dessin annexé dans lequel :
- la figure 1 est une vue en coupe axiale d'un dispositif de sécurité selon l'invention, renfermant une seringue,
- la figure 2 est une vue analogue à la figure 1 montrant la seringue en position de sécurité,
- la figure 3 est une vue à plus grande échelle du détail III de la figure 2.

La seringue comprend un corps de seringue 1 muni à son extrémité avant d'un porte-aiguille 2 sur lequel est montée une aiguille 2'.

A son extrémité arrière, le corps de la seringue comporte une collerette 3.

Un piston 4 est mobile à l'intérieur du corps de la seringue et sert à injecter le produit contenu dans le corps de la seringue, sous l'action d'une tige de piston 5 dépassant de l'extrémité arrière du corps de la seringue et terminée par un poussoir 6 sur lequel l'utilisateur de la seringue exerce une pression pour injecter le produit.

La seringue est placée dans un dispositif de sécurité comprenant un fourreau constitué par une partie cylindrique 7 ayant une extrémité avant 8 et une tête 9 opposée à l'extrémité 8.

La tête 9 est réalisée en deux parties dont l'une est mobile axialement de sorte que la tête 9 peut être qualifiée de télescopique.

La partie fixe de la tête 9 comprend une portion tubulaire 10 qui est intérieurement plus large que la partie cylindrique 7 du fourreau et loge un ressort hélicoïdal 11 qui définit intérieurement un volume cylindrique prolongeant la cavité intérieure du fourreau, de sorte que le corps 1 de la seringue engagé dans le fourreau traverse axialement le ressort hélicoïdal 11 sans difficulté.

La collerette 3 maintient le ressort à l'état comprimé.

La partie fixe de la tête 9 comprend également une paroi extérieure 12 qui délimite, autour de la portion tubulaire 10, une chambre annulaire 13.

La paroi 12 aboutit à une bague 14 qui, pour des raisons de fabrication, a été réalisée séparément puis rapportée sur le fourreau mais pourrait être réalisée d'un seul tenant avec lui.

La bague 14 comporte un passage central 15 formé en goulot et supporte une patte élastique 16 qui sert de butée axiale à la collerette 3 du corps 1 de la seringue, en maintenant ce dernier en position d'injection dans le fourreau, le ressort 11 étant comprimé dans son logement.

Dans cette position d'injection, on voit que le porte-aiguille 2 affleure à l'extrémité inférieure 8 du fourreau tandis que le corps 1 de la seringue est totalement compris dans le fourreau.

La partie mobile de la tête 9 est constituée par un capuchon 17 de forme générale cylindrique.

Le capuchon 17 comporte un perçage central 18 qui permet le passage de la tige de piston 5 et une encoche longitudinale 19 partant de son bord inférieur 20 et permettant au capuchon 17 de s'engager dans la chambre annulaire 13 de part et d'autre de la patte élastique 16.

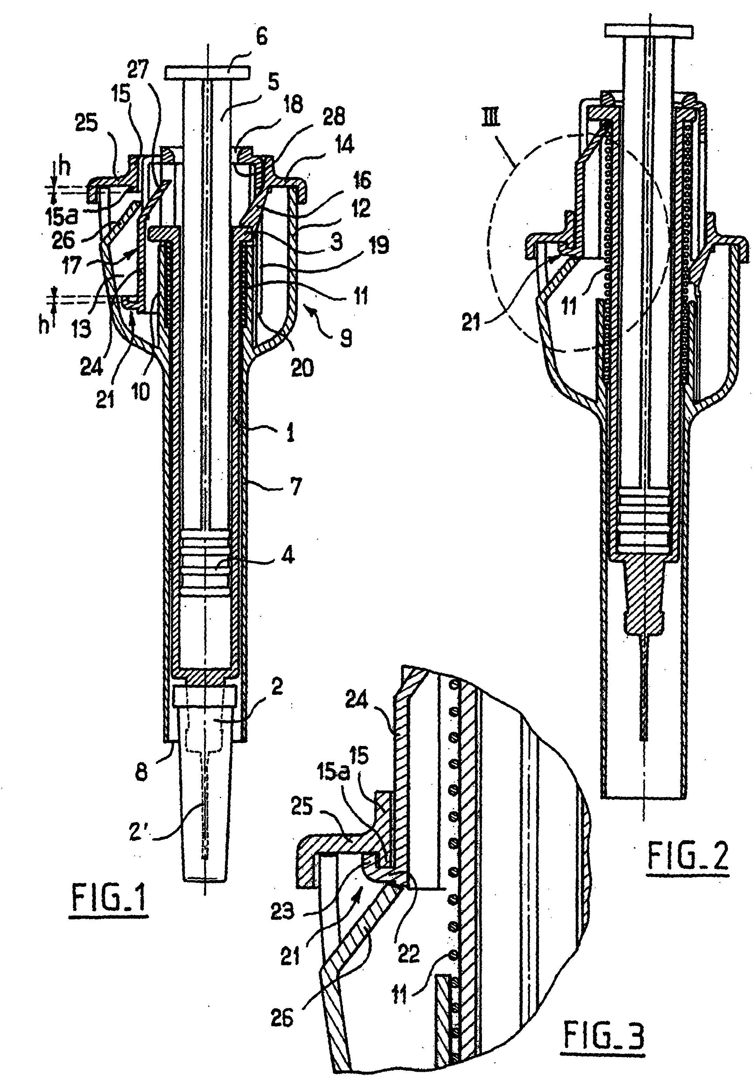

En dehors de l'encoche 19, le capuchon comporte, à son extrémité inférieure 20, un rebord 21 dont le détail est mieux visible sur la figure 3.

Le rebord 21 est constitué par une première portion de paroi 22 qui s'étend radialement vers l'extérieur à la manière d'une collerette et une deuxième portion de paroi 23 qui s'étend axialement vers le haut, c'est à dire parallèlement à la paroi 24 du capuchon, en direction opposée à l'extrémité inférieure 20 du capuchon.

La paroi radiale 22 se situe à distance de l'extrémité inférieure 20 du capuchon de sorte qu'un angle se forme entre la face inférieure de la paroi radiale 22 et la face extérieure de la paroi latérale 24.

Le diamètre extérieur du capuchon 17 est ajusté au diamètre intérieur du goulot 15, lequel se prolonge vers le bas en dessous de la paroi de fermeture 25 de la bague 14, par une portion 15a de hauteur h sensiblement égale à celle de la paroi latérale 23.

A l'opposé de la patte élastique 16, la paroi 12 de la tête 9 comporte une patte élastique 26 qui s'étend vers l'intérieur de la tête jusqu'en regard de l'extrémité inférieure de la portion 15a du goulot 15, en étant située à une distance de celle-ci correspondant sensiblement à l'épaisseur de la paroi radiale 22.

Le fonctionnement du dispositif de sécurité est le suivant.

La seringue se trouvant en position d'injection, comme représenté à la figure 1, l'utilisateur peut enfoncer la tige de piston 5 en exerçant une pression sur le poussoir 6.

Lorsqu'il arrive presque en fin de course, le piston 4 se trouve au voisinage du porte-aiguille 2 et le poussoir 6 est au contact du capuchon 17.

La fin de course du piston entraîne le capuchon 17 vers le bas, ce qui provoque son enfoncement dans la tête 9.

L'encoche 19 ne s'étendant pas sur toute la hauteur du capuchon, la paroi latérale de ce dernier écarte la patte élastique 16, ce qui libère le corps de la seringue qui peut ainsi remonter sous l'action du ressort hélicoïdal 11.

Ce mouvement de remontée ne se produit cependant pas tant que l'utilisateur maintient une pression suffisante sur le poussoir 6.

Lorsque l'utilisateur relâche la pression, le corps de la seringue remonte sous l'action du ressort.

Dans un premier temps, la collerette 3 repousse le capuchon vers le haut en prenant appui sur une patte élastique 27 dirigée vers l'intérieur.

A mesure que le corps de la seringue continue sa progression vers le haut, le capuchon remonte et la paroi latérale 23 arrive au contact de la patte élastique 26 de la tête.

Sous l'action du ressort, la patte élastique 26 s'escamote et laisse les parois latérale 23 et radiale 22 arriver au contact de la portion 15a du goulot 15.

Le capuchon est ainsi arrivé en fin de course et a atteint sa position sortie.

Le corps de la seringue termine sa course vers le haut lorsque la collerette 3 écarte la patte élastique 27 et vient s'appuyer contre la paroi supérieure 28 du capuchon.

La patte élastique 27 revient alors en position et se loge sous la collerette 3, immobilisant ainsi le corps 1 de la seringue par rapport au capuchon.

Ce dernier étant immobilisé par rapport au fourreau, comme cela va être détaillé, le corps de la seringue se trouve immobilisé en position de sécurité dans le fourreau, le porte-aiguille se trouvant en retrait dans le fourreau et l'aiguille ne dépassant pas de l'extrémité inférieure 8 du fourreau.

L'aiguille ainsi rétractée préserve les utilisateurs de tout risque de contact ou de piqûre avec elle.

Il est à noter que, selon la raideur des pattes élastiques 26 et 27, il est possible, contrairement à ce qui vient d'être décrit, que, dans un premier temps, la collerette 3 passe au-dessus de la patte élastique 27 et que, dans un second temps, les parois latérales 23 et radiales 22 passent au-dessus de la patte élastique 26.

On va maintenant détailler l'immobilisation axiale réalisée entre le capuchon et le fourreau.

Comme on le voit sur la figure 3, la distance entre l'extrémité inférieure du goulot 15 et l'extrémité de la patte élastique 26 correspond sensiblement à l'épaisseur de la paroi radiale 22.

Par conséquent, cette dernière est coincée entre le goulot et la patte élastique 26, laquelle se loge dans l'angle formé sous la paroi 22.

En d'autres termes, les faces supérieure et inférieure de la paroi radiale 22 constituent deux butées axiales opposées qui s'appuient sur deux butées axiales du fourreau constituées par l'extrémité inférieure du goulot 15 et par l'extrémité de la patte élastique 26.

Par ailleurs, la distance entre les parois latérales 23 et 24 correspond sensiblement à l'épaisseur de la portion 15a du goulot 15, de sorte que la face interne de la paroi latérale 23 et la face externe de la paroi latérale 24 constituent des butées latérales opposées qui s'appuient sur des butées latérales opposées du fourreau constituées par les faces interne et externe de la portion 15a du goulot 15.

Ainsi, les butées radiales et axiales mentionnées ci-dessus se combinent entre elles pour assurer une solidarisation fiable du capuchon 17 dans la tête sur le fourreau.

De cette manière, le dispositif selon l'invention peut subir différentes pressions radiales et axiales sans que cela ne modifie le verrouillage du capuchon sur le fourreau et, par conséquent, sans que cela ne remette en question la protection procurée par le dispositif lorsque le corps de la seringue se trouve en position de sécurité.

Il est à noter que le dispositif selon l'invention est agencé pour pouvoir être utilisé avec une seringue de type traditionnel, sans que cette dernière n'ait à subir aucune modification de sa géométrie.

Il est bien entendu que le mode de réalisation qui vient d'être décrit ne présente aucun caractère limitatif et pourra recevoir toutes modifications désirables sans sortir pour cela du cadre de l'invention.

## Revendications

1. Dispositif de sécurité pour une seringue constitué d'un corps (1), d'un porte-aiguille (2) monté à une extrémité de ce corps, d'un piston (4) mobile dans le corps et d'une tige de piston (5) dépassant du corps à l'opposé du porte-aiguille et apte à pousser le piston dans le corps en direction du porte-aiguille, le dispositif comprenant un fourreau (7) ayant une extrémité avant (8) et une extrémité arrière, dans lequel un corps de seringue peut coulisser axialement entre une position d'injection dans laquelle le corps de la seringue est intégralement contenu dans le fourreau, tandis que le porte-aiguille de la seringue affleure à l'extrémité avant du fourreau, et une position de sécurité dans laquelle une partie du corps de la seringue dépasse de l'extrémité arrière du fourreau, tandis que le porte-aiguille se trouve en retrait de l'extrémité avant du fourreau, le dispositif comprenant également un organe de rappel (11) du corps de seringue en position de sécurité, lequel organe de rappel se déclenche automatiquement lorsque l'on enfonce la tige de piston dans le corps de la seringue, **caractérisé par le fait que** le fourreau comporte, à son extrémité arrière, une tête télescopique (9) pouvant prendre une position rentrée dans laquelle elle n'agit pas sur le corps de la seringue en position d'injection et une position sortie dans laquelle elle enveloppe la partie du corps de la seringue en position de sécurité dépassant de l'extrémité arrière du fourreau, le dispositif comportant en outre des moyens de verrouillage de la tête télescopique en position sortie, constitués par une combinaison de deux portées d'appui (22) axiales opposées de la tête télescopique qui s'appuient sur deux portées d'appui (15a, 26) axiales opposées du fourreau et de deux portées d'appui (23, 24) latérales opposées de la tête télescopique qui s'appuient sur deux portées d'appui (15a) latérales opposées du fourreau.

2. Dispositif selon la revendication 1 **caractérisé par le fait que** la tête télescopique (9) réalise l'immobilisation axiale du corps de la seringue par rapport au fourreau.

3. Dispositif selon la revendication 1 **caractérisé par le fait que** le fourreau comporte une portée d'appui pour immobiliser le corps de la seringue en position de sécurité.

4. Dispositif selon l'une quelconque des revendications 1 à 3 **caractérisé par le fait que** la tête du fourreau sert à déclencher l'organe de rappel (11) lorsque l'on enfonce la tige de piston dans le corps de la seringue.

5. Dispositif selon l'une quelconque des revendications 1 à 4 **caractérisé par le fait que** les deux butées axiales opposées de la tête sont constituées par les faces supérieure et inférieure d'une paroi radiale (22) qui s'étend vers l'extérieur à la manière d'une collerette à partir de la paroi latérale (24) d'un capuchon (17) constituant la partie mobile de la tête télescopique (9) et que les deux butées latérales opposées de la tête sont constituées par la face interne d'une paroi latérale (23) s'étendant à partir de la paroi radiale (22) et par la face externe de la paroi latérale (24) du capuchon.

6. Dispositif selon la revendication précédente **caractérisé par le fait que** les butées latérales opposées du fourreau sont constituées par les faces internes et externes de l'extrémité inférieure (15a) d'un goulot (15) dans lequel coulisse le capuchon mobile (17) et que les butées axiales opposées du fourreau sont constituées par l'extrémité inférieure du goulot (15) et par l'extrémité d'une patte élastique (26) situé en regard de ladite extrémité inférieure du goulot.

## Patentansprüche

1. Schutzvorrichtung für eine Spritze, bestehend aus einem Körper (1), einem an einem Ende dieses Körpers befestigten Nadelhalter (2), einem im Körper beweglichen Kolben und einer gegenüber dem Nadelhalter über den Körper überstehenden Kolbenstange (5), die in der Lage ist, den Kolben in den Körper in Richtung des Nadelhalters zu drücken, wobei diese Vorrichtung eine Hülse (7) mit einem vorderen Ende (8) und einem hinteren Ende aufweist, in die der Spritzenkörper axial zwischen einer Injektionsstellung, in der der Körper der Spritze sich vollständig in der Hülse befindet, während der Nadelhalter der Spritze das vordere Ende der Hülse erreicht, und einer Schutzstellung, in der ein Teil des Körper der Spritze über das hintere Ende der Hülse herausragt, während der Spritzenhalter sich im Rücksprung zum vorderen Ende der Hülse befindet, und die Vorrichtung ferner ein Mitnahmeglied (11) des Spritzenkörpers in der Schutzstellung aufweist, dass automatisch ausgelöst wird, wenn man die Kolbenstange in den Körper der Spritze drückt, **dadurch gekennzeichnet, dass** am hinteren Ende der Hülse ein Teleskopkopf (9) angeordnet wird, der eine Einzugsstellung, in der er nicht auf den Körper der Spritze in Injektionsstellung.einwirkt, und eine Auszugsstellung, in der er den über das hintere Ende der Hülse überstehende Teil des Körpers der Spritz in Schutzstellung umhüllt, wobei die Vorrichtung darüber hinaus mit Verriegelungsmitteln des Teleskopkopfs in Auszugsstellung versehen ist, die durch eine Verbindung von zwei dem Teleskopkopf axial gegenüber liegenden Stützzapfen (22), die sich auf zwei der Hülse axial gegenüber liegende Stützzapfen (15a, 26) stützen, und von zwei dem Teleskopkopf lateral gegenüber liegenden Stützzapfen (23, 24), die sich auf zwei der Hülse lateral gegenüber liegende Stützzapfen (15a) stützen, gebildet werden.

2. Vorrichtung nach Anspruch 1, **dadurch gekennzeichnet, dass** der Teleskopkopf (9) die Blockierung des Körpers der Spritze axial zur Hülse bewirkt.

3. Vorrichtung nach Anspruch 1, **dadurch gekennzeichnet, dass** die Hülse einen Stützzapfen zur Blockierung des Körpers der Spritz in der Schutzstellung aufweist.

4. Vorrichtung nach einem der Ansprüche 1 bis 3, **dadurch gekennzeichnet, dass** der Kopf der Hülse die Auslösung des Mitnahmeglieds (11) bewirkt, wenn die Kolbenstange in den Körper der Spritze gedrückt wird.

5. Vorrichtung nach einem der Ansprüche 1 bis 4, **dadurch gekennzeichnet, dass** die beiden dem Kopf axial gegenüber liegenden Anschläge durch Ober- und Unterseiten einer Radialwand (22) gebildet werden, die ab der Seitenwand (24) eines den mobilen Teil des Teleskopkopfes (9) bildenden Deckels (17) wie ein Kragen nach außen ausläuft und die beiden dem Kopf lateral gegenüber liegenden Anschläge aus der Innenseite einer sich ab der Radialwand erstreckenden Seitenwand (23) und aus der Außenseite der Seitenwand (24) des Deckels gebildet werden.

6. Vorrichtung nach vorstehendem Anspruch, **dadurch gekennzeichnet, dass** die der Hülse lateral gegenüber liegenden Anschläge aus der Innen- und Außenseite des unteren Endes (15a) eines Stutzens (15) gebildet werden, in dem der mobile Deckel (17) gleitet, und die der Hülse axial gegenüber liegenden Anschläge aus dem unteren Ende des Stutzens und der Außenseite einer Federklammer (26) gebildet werden, die gegenüber diesem unteren Ende des Stutzens angeordnet ist.

## Claims

1. A safety device for a syringe constituted by a body (1), a needle carrier (2) mounted at one end of the body, a piston (4) movable inside the body, and a piston plunger (5) projecting from the body at its end opposite from the needle carrier and suitable for pushing the piston into the body towards the needle carrier, the device comprising a sheath (7) having a front end (8) and a rear end, the syringe body being capable of sliding axially inside the sheath between an injection position in which the syringe body is fully contained within the sheath while the needle carrier of the syringe is flush with the front end of the sheath, and a safe position in which a portion of the syringe body projects from the rear end of the sheath while the needle carrier is set back from the front end of the sheath, the device also comprising a return member (11) urging the syringe body towards the safe position, which return member is triggered automatically when the piston plunger is pushed into the body of the syringe, **characterized by** the fact that the rear end of the sheath has a telescopic head (9) capable of taking up a retracted position in which it does not act on the syringe body when in the injection position, and an extended position in which it surrounds the portion of the syringe body that projects from the rear end of the sheath when in the safe position, the device further comprising locking means for locking the telescopic head in the extended position, said locking means being constituted by a combination of two oppositely-directed axial bearing surfaces (22) of the telescopic head which bear against two oppositely-directed axial bearing surfaces (15a, 26) of the sheath, and of two oppositely-directed lateral bearing surfaces (23, 24) of the telescopic head which bear against two oppositely-directed lateral bearing surfaces (15a) of the sheath.

2. A device according to claim 1, **characterized by** the fact that the telescopic head (9) prevents the syringe body from moving axially relative to the sheath.

3. A device according to claim 1, **characterized by** the fact that the sheath has a bearing surface for preventing the syringe body from moving when in the safe position.

4. A device according to any one of claims 1 to 3, **characterized by** the fact that the head of the sheath serves to trigger the return member (11) when the piston plunger is pushed into the body of the syringe.

5. A device according to any one of claims 1 to 4, **characterized by** the facts that the two oppositely-directed axial bearing surfaces of the head are constituted by the top and bottom faces of a radial wall (22) that extend outwards so as to form a collar on a side wall (24) of a cap (17) constituting the moving portion of the telescopic head (9), and that the two oppositely-directed lateral abutment surfaces of the head are constituted by an inner face of a side wall (23) extending from the radial wall (22) and by the outer face of the side wall (24) of the cap.

6. A device according to the preceding claim, **characterized by** the facts that the oppositely-directed lateral abutment surfaces of the sheath are constituted by the inner and outer faces of the bottom end (15a) of a neck (15) in which the moving cap (17) slides, and that the oppositely-directed axial abutment surfaces of the sheath are constituted by the bottom end of the neck (15) and by the end of a resilient tab (26) situated facing said bottom end of the neck.
